# EUROPÄISCHE PATENTSCHRIFT

(11) **EP 3 396 092 B1**
(45) Veröffentlichungstag und Bekanntmachung des Hinweises auf die Patenterteilung: **05.05.2021**
(21) Anmeldenummer: 18162431.3
(22) Anmeldetag: 16.03.2018
(51) Int. Cl.: E05C 7/02, F25D 23/02, E05B 47/02

(54) **LABORSCHRANK**
LABORATORY CABINET
ARMOIRE DE LABORATOIRE

(30) Priorität: 28.04.2017 DE 102017109263
(43) Veröffentlichungstag der Anmeldung: 31.10.2018
(73) Patentinhaber: Binder GmbH, 78532 Tuttlingen (DE)
(72) Erfinder: Baumgärtner, Eugen, 78050 Villingen-Schwenningen (DE); Efinger, Patrick, 78582 Balgheim (DE); Storz, Ewald, 78604 Rietheim-Weilheim (DE); Dr. Winkler, Hartmut, 78532 Tuttlingen (DE); Leidolt, Richard, 78224 Singen (DE); Binder, Peter Michael, 8595 Altnau (CH)
(74) Vertreter: Westphal, Mussgnug & Partner Patentanwälte mbB

(56) Entgegenhaltungen:
- EP-A2- 1 977 176
- EP-A2- 2 565 565
- WO-A1-2015/072793
- WO-A1-2015/137725
- DE-C1- 19 803 601
- US-A- 2 204 053
- US-A- 3 086 830

## Beschreibung

Die Erfindung betrifft einen Laborschrank gemäß Patentanspruch 1.

Bekannt sind Laborschränke mit einem wenigstens eine Außentür mit einem Außentürverschluss aufweisenden Gehäuse, welches wenigstens einen Innenraum aufweist. Oft weisen derartige Laborschränke Vorrichtungen auf, mit welchen im Innenraum eine definierte Temperatur und/oder definierte Klimabedingungen, beispielsweise eine definierte Feuchte, einstellbar sind. Beispielsweise sind Kälteschränke, Wärmeschränke, Trockenschränke oder Brutschränke bekannt. Um zu verhindern, dass beim Öffnen der Außentür die klimatischen Bedingungen im Innenraum gestört werden, ist es bekannt, den Innenraum durch eine Innentür mit einem Innentürverschluss zu verschließen. Die Außentüre dient im Wesentlichen der Dämmung zwischen den Innenraum des Laborschranks und der Umgebung. Die Innentür verhindert den Luftaustausch des Innenraums mit der Umgebung beim Öffnen der Außentüre. Die Innentür kann aus transparentem Material gefertigt sein, was es ermöglicht, bei geöffneter Außentür einen Blick in den Innenraum zu werden, ohne die klimatischen Bedingungen im Innenraum zu stören.

Bekannt sind auch Laborschränke, bei welchen der Innenraum in mehrere Teilräume aufgeteilt ist und mehrere Innentüren die kleineren Teilräume verschließen, so dass das Klima in den verbleibenden Teilräumen möglichst wenig gestört wird, wenn in einen der Teilräume Probengut eingelagert wird oder aus dem Teilraum Probengut entnommen wird.

Bei den bekannten Laborschränken ist es erforderlich, dass ein Benutzer zunächst die Außentür des Laborschranks öffnet und anschließend mit einem zweiten Handgriff die Innentür öffnen kann.

Weiterer Stand der Technik bildet die Druckschrift EP 2 565 565 A2, die einen Kühlschrank mit einer Außentür und einer Innentür lehrt. Die Innentür weist einen Innentürverschluss auf, der durch betätigbare Mittel aktiviert werden kann, damit bei Aufziehen der Außentür die Innentür verriegelt wird und nicht mit der Außentür öffnet.

Die Aufgabe der Erfindung besteht darin, einen Laborschrank dahingehend weiterzubilden, dass er benutzerfreundlicher zu handhaben ist und insbesondere das Öffnen der Innentür vereinfacht wird.

Die Aufgabe der Erfindung wird gelöst durch einen Laborschrank mit den Merkmalen des Patentanspruchs 1.

Vorteilhafte Ausgestaltungen und Weiterbildungen der Erfindung sind in den abhängigen Ansprüchen angegeben.

Der erfindungsgemäße Laborschrank, beispielsweise Kälteschrank, Wärmeschrank, Trockenschrank oder Brutschrank, mit einem wenigstens eine Außentür mit einem Außentürverschluss aufweisenden Gehäuse, welches wenigstens einen Innenraum aufweist, der durch eine Innentür mit einem Innentürverschluss verschließbar ist, zeichnet sich dadurch aus, dass der Innentürverschluss ein mechanischer Verschluss ist, dass der Laborschrank bei geschlossener Außentür betätigbare erste Mittel zum Entriegeln des Innentürverschlusses der Innentür aufweist, welche elektrisch ansteuerbar sind, und dass der Laborschrank zweite Mittel aufweist, welche derart ausgebildet sind, dass beim Öffnen der Außentür die Innentür ebenfalls öffnet. Ein derartig ausgestalteter Laborschrank ermöglicht es dem Benutzer, bereits vor dem Öffnen der Außentür die Innentür zu entriegeln, und bewirkt, dass beim Öffnen der Außentür auch die Innentür bereits geöffnet wird, so dass der zusätzliche Handgriff, mit welchem die Innentür nach dem Öffnen der Außentür separat geöffnet werden muss, entfallen kann.

Unter einem Verschluss soll vorliegend derjenige Mechanismus verstanden werden, welcher die entsprechende Türe gegen zufälliges Verschwenken sichert. Es kann an jedem der Verschlüsse zusätzlich ein Sperrmechanismus vorgesehen sein, welcher verhindert, dass der Verschluss geöffnet werden kann.

Gemäß einer vorteilhaften Ausgestaltung der Erfindung weist der mechanische Verschluss einen Permanentmagneten, einen Klemmmechanismus, einen Rastmechanismus, einen Federmechanismus, einen Schnappmechanismus und/oder einen Kugelschnappmechanismus auf. Derartige Verschlüsse sind insbesondere bei hohen oder tiefen Temperaturen oder großer Feuchte wenig störanfällig.

Erfindungsgemäß ist vorgesehen, dass die ersten Mittel als an einer von Außentür und Innentür angeordneter Elektromagnet ausgebildet ist, welcher im aktivierten Zustand mit dem anderen von Außentür und Innentür oder mit einem an dem anderen von Außentür und Innentür angeordneten magnetisierbaren Element zusammenwirkt und dessen Haltekraft größer ist als die Verschlusskraft des mechanischen Verschlusses. Mit anderen Worten sind die ersten Mittel als Elektromagnet ausgebildet, welcher entweder an der Außentür oder der Innentür angeordnet ist. Im aktivierten Zustand wirkt der Elektromagnet, wenn er an der Außentür angeordnet ist, mit der Innentür oder einem an der Innentür angeordneten magnetisierbaren Element zusammen, während er, wenn er an der Innentür angeordnet ist, mit der Außentür oder einem an der Außentür angeordneten magnetisierbaren Element zusammenwirkt. Der Elektromagnet stellt somit eine Kopplung zwischen der Außentür und der Innentür her. Dadurch, dass die Haltekraft des Elektromagneten größer ist als die Verschlusskraft des mechanischen Verschlusses, wird der mechanische Verschluss der Innentür gegen die Verschlusskraft geöffnet, wenn die Außentür geöffnet wird, und somit entriegelt. Ist der Elektromagnet auch während des weiteren Öffnungsvorgangs der Außentür aktiviert, folgt die Innentür der Außentür gehalten durch den Elektromagneten nach, so dass in diesem Fall der Elektromagnet nicht nur die elektrisch ansteuerbaren ersten Mittel zum Entriegeln des Innentürverschlusses der Innentür, sondern gleichzeitig auch die zweiten Mittel bilden kann.

Gemäß einer vorteilhaften Ausgestaltung der Erfindung sind die ersten Mittel als insbesondere an einer von Außentür und Innentür angeordneter Elektromagnet ausgebildet, welcher im aktivierten Zustand den mechanischen Verschluss entriegelt. Ein Elektromagnet ist bei extremen klimatischen Bedingungen wenig störanfällig.

Vorteilhafterweise entriegelt der Elektromagnet im aktivierten Zustand den mechanischen Verschluss durch Bewegen eines Elements des mechanischen Verschlusses, beispielsweise durch Verschieben eines Riegels und/oder Verschieben oder Freigeben eines Rastelements. Eine derartige Entriegelung ist wenig störanfällig, insbesondere bei extremen Temperaturen, besonders bei extrem tiefen Temperaturen.

Gemäß einer vorteilhaften Weiterbildung der Erfindung weist der mechanische Verschluss einen Schnappverschluss mit einem federbelasteten Vorsprung auf, wobei der Elektromagnet im aktivierten Zustand den Vorsprung insbesondere über einen Hebelmechanismus, beispielsweise ein Hebelgestänge, gegen die Federkraft zurückzieht, um den Schnappverschluss zu entriegeln. Eine derartige Entriegelung ist wenig störanfällig, insbesondere bei extremen Temperaturen, besonders bei extrem tiefen Temperaturen.

Eine vorteilhafte Weiterbildung der Erfindung sieht vor, dass die ersten Mittel als magnetisch erregbare Schubstange ausgebildet sind und der mechanische Verschluss einen Rastmechanismus mit einem federbelasteten Riegel mit einer Öffnung aufweist, in welche ein Rastkopf eingreift, wobei der Elektromagnet im aktivierten Zustand den Riegel gegen die Federkraft bewegt, um den Rastverschluss zu entriegeln. Eine derartige Entriegelung ist wenig störanfällig.

Gemäß einer bevorzugten Ausführungsform der Erfindung sind die ersten Mittel als elektromagnetisch erregbare Schubstange ausgebildet, welche im ausgefahrenen Zustand ein an dem anderen von Außentür und Innentür angeordnetes Element hintergreift. Auf diese Weise kann eine Kopplung zwischen der Innentür und der Außentür erreicht werden, welche die Verschlusskraft des Innentürverschlusses überwinden kann.

Die zweiten Mittel sind gemäß einer bevorzugten Ausführungsform der Erfindung als zwischen der Außentür und der Innentür angeordneter Mitnehmer ausgebildet. Ein Mitnehmer bewirkt dabei eine Kopplung zwischen Außentür und Innentür, die zur Folge hat, dass beim Öffnen der Außentür die Innentür mitgenommen, insbesondere mitgezogen, wird.

Gemäß einer bevorzugten Ausführungsform der Erfindung ist der Mitnehmer als an einer von Außentür und Innentür angeordneter Permanentmagnet oder Elektromagnet ausgebildet, welcher mit dem anderen von Außentür und Innentür oder mit einem an dem anderen von Außentür und Innentür angeordneten magnetisierbaren Element zusammenwirkt. Durch einen derartig ausgestalteten Mitnehmer ergibt sich eine magnetische Kopplung zwischen Innentür und Außentür, welche vorteilhafterweise auch eine Relativbewegung zwischen Innentür und Außentür beim Schwenken der beiden Türen um unterschiedliche Drehpunkte ermöglicht.

Eine bevorzugte Ausführungsform der Erfindung sieht vor, dass der Mitnehmer als an einer von Außentür und Innentür angeordneter elektrisch ausfahrbarer Stößel oder Riegel oder elektromagnetisch erregbare Schubstange ausgebildet ist, welcher oder welche im ausgefahrenen Zustand ein an dem anderen von Außentür und Innentür angeordnetes Element hintergreift. Ein derartiger Mitnehmer erlaubt eine zuverlässige Kopplung, wobei in Abhängigkeit von der Ausgestaltung des hintergriffenen Elements auch eine Relativbewegung zwischen Außentür und Innentür, welche durch die an unterschiedlichen Positionen angeordneten Angelpunkte der Türen hervorgerufen wird, ermöglicht wird.

Gemäß einer vorteilhaften Ausführungsform der Erfindung sind die zweiten Mittel als die Innentür beaufschlagende Feder, beispielsweise als Torsionsfeder, ausgebildet. Derartige ausgebildete zweite Mittel bewirken, dass bei Öffnen der Außentür die Innentür federbelastet aufspringt, ohne dass eine sonstige Kopplung zwischen Innentür und Außentür erforderlich ist. Die Innentür und die Außentür bleiben dabei unabhängig voneinander verschwenkbar.

Gemäß einer besonders bevorzugten Ausführungsform der Erfindung bilden die ersten Mittel gleichzeitig die zweiten Mittel, was den Vorteil aufweist, das möglichst wenig zusätzliche Komponenten an dem Laborschrank erforderlich sind.

Vorteilhafterweise ist der Innentürverschluss bei geöffneter Außentür manuell entriegelbar, was es dem Benutzer erlaubt, auch nach Öffnung der Außentür die Innentür zu öffnen oder bei Vorhandensein mehrerer Innentüren nach Öffnung der Außentür eine oder weitere Innentüren ergänzend zu öffnen.

Eine besonders bevorzugte Ausführungsform der Erfindung sieht vor, dass an der Außentür oder der Innentür ein Dämpfungselement, beispielsweise ein Federelement oder ein elastisches Element, angeordnet ist, welches bei einer Schließbewegung der Außentür zwischen der Außentür und der Innentür zur Anlage kommt. Das Dämpfungselement bewirkt beim Schließen der Außentür vorteilhafterweise eine Beaufschlagung der Innentür derart, dass der Innentürverschluss wieder eingreift. Dadurch wird es ermöglicht, die Innentür gleichzeitig mit der Außentür wieder zu verschließen, ohne dass ein separater Schließvorgang für die Innentür alleine notwendig ist.

Eine bevorzugte Ausführungsform der Erfindung sieht vor, dass die Innentür zumindest abschnittsweise aus transparentem Material, insbesondere Glas, gefertigt ist. Dies ermöglicht eine Einsicht in den Innenraum, ohne dass die Innentür geöffnet wird und damit die klimatischen Bedingungen im Innenraum gestört werden.

Eine vorteilhafte Weiterbildung der Erfindung sieht vor, dass der Laborschrank mehrere Innentüren aufweist und die ersten Mittel derart ausgebildet sind, dass durch die ersten Mittel eine oder mehrere der Innentüren entriegelbar sind. Eine derartige Ausgestaltung ermöglicht dem Benutzer eine Vorwahl an der Außenseite des Gehäuses derart, dass er vor dem Öffnen der Außentür auswählen kann, welche Innentür oder welche Innentüren gleichzeitig mit der Außentür geöffnet werden sollen. Dabei besteht vorteilhafterweise zusätzlich die Möglichkeit, auch nicht ausgewählte Innentüren nach dem Öffnen der Außentür zusätzlich manuell zu öffnen, falls dies erforderlich sein sollte.

Die Erfindung wird anhand der nachfolgenden Figuren ausführlich erläutert. Es zeigen
- Figur 1: eine perspektivische Ansicht eines Ausführungsbeispiels eines Laborschranks mit einem ersten Ausführungsbeispiel eines Verschluss- und Entriegelungsmechanismus mit geöffneter Außentür,
- Figur 2: eine perspektivische Ansicht des Laborschranks gemäß Figur 1 mit geöffneter Außentür und geöffneter Innentür,
- Figur 3: eine weitere perspektivische Ansicht des Laborschranks gemäß Figur 2,
- Figur 4: einen Ausschnitt aus einem Querschnitt durch ein weiteres Ausführungsbeispiel eines Laborschranks mit einem zweiten Ausführungsbeispiel eines Verschluss- und Entriegelungsmechanismus,
- Figur 5: einen Ausschnitt aus einem Querschnitt durch ein weiteres Ausführungsbeispiel eines Laborschranks mit einem dritten Ausführungsbeispiel eines Verschluss- und Entriegelungsmechanismus,
- Figur 6: einen Ausschnitt aus einem Querschnitt durch ein weiteres Ausführungsbeispiel eines Laborschranks mit einem vierten Ausführungsbeispiel eines Verschlussund Entriegelungsmechanismus,
- Figur 7: einen Ausschnitt aus einem Querschnitt durch ein weiteres Ausführungsbeispiel eines Laborschranks mit einem fünften Ausführungsbeispiel eines Verschluss- und Entriegelungsmechanismus,
- Figur 8: einen Ausschnitt aus einem Querschnitt durch ein weiteres Ausführungsbeispiel eines Laborschranks mit einem sechsten Ausführungsbeispiel eines Verschlusses,
- Figur 9: einen Ausschnitt aus einem Querschnitt durch ein weiteres Ausführungsbeispiel eines Laborschranks mit einem siebten Ausführungsbeispiel eines Verschlussund Entriegelungsmechanismus,
- Figur 10: einen Längsschnitt durch den Verschluss- und Entriegelungsmechanismus des Laborschranks gemäß Figur 1 mit geschlossener Innentür und geschlossener Außentür,
- Figur 11: eine perspektivische Ansicht des Innentürverschlusses des Laborschranks gemäß Figur 1,
- Figur 12: eine Rückansicht des Innentürverschlusses gemäß Figur 11,
- Figur 13: einen Längsschnitt durch den Innentürverschluss gemäß Figur 11,
- Figur 14: einen Längsschnitt durch den am Rahmen montierten Teil des Innentürverschlusses gemäß Figur 11 und
- Figur 15: eine Explosionsdarstellung des am Rahmen montierten Teils des Innentürverschlusses gemäß Figur 11.

Gleiche Bezugsziffern bezeichnen auch in unterschiedlichen Ausführungsbeispielen gleiche oder gleichartige Teile. Zur besseren Übersicht sind nicht sämtliche Bezugsziffern in sämtlichen Figuren angegeben.

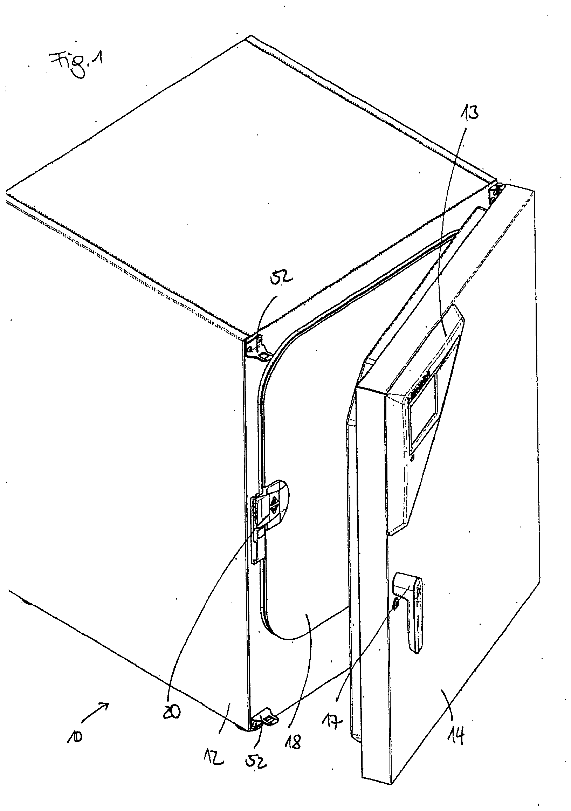

Die Figuren 1 bis 3 zeigen verschiedene perspektivische Ansichten eines Ausführungsbeispiels eines Laborschranks 10, welcher beispielsweise als Kälteschrank, Wärmeschrank, Trockenschrank oder Brutschrank ausgebildet sein kann. Der Laborschrank 10 weist ein Gehäuse 12 mit einer Außentür 14 auf, welches einen Innenraum umschließt. Das Gehäuse 12 kann eine Bodenseite, eine Deckseite, zwei gegenüberliegende Seitenwände und eine Rückwand aufweisen, wobei eine der Rückwand gegenüberliegende Öffnung durch die Außentür 14 verschlossen werden kann. Die Außentür 14 kann über einen Außentürverschluss 16 geschlossen werden, welcher durch einen Handgriff 17 betätigt werden kann. Der Außentürverschluss 16 sichert die Außentür 14 gegen zufälliges Verschwenken. Es kann ein zusätzlicher Sperrmechanismus vorgesehen sein, welcher das Entriegeln des Außentürverschlusses verhindert.

Das Gehäuse 12 kann zur besseren Wärmedämmung doppelwandig ausgebildet sein und insbesondere einen Innenkessel aufweisen. Der Laborschrank 10 weist wenigstens eine Innentür 18 auf, welche eine zur Außentür 14 weisende Öffnung des Innenraums verschließt, um einen Luftaustausch des Innenraums mit der Umgebung zu unterbinden oder zu verringern, wenn die Außentür 14 geöffnet wird.

Die Innentür 18 weist einen Innentürverschluss 20 auf, welcher die Innentür 18 in einer geschlossenen Position gegen Verschwenken sichert. Zusätzlich kann die Innentür 18 einen Sperrmechanismus aufweisen, welcher verhindert, dass der Innentürverschluss 20 geöffnet werden kann.

Die Innentür 18 kann zumindest abschnittsweise oder auch vollständig aus einem transparenten Material, beispielsweise Glas, gefertigt sein.

Es ist möglich, dass der Innenraum in mehrere Teilräume unterteilt ist, welche jeweils durch eine separate Innentür 18 verschlossen sind, so dass lediglich eine der Innentüren 18 geöffnet werden muss, wenn Probengut in den Laborschrank 10, insbesondere in einen der Teilräume, eingelagert oder aus dem Laborschrank 10 entnommen werden soll, dabei aber die klimatischen Bedingungen in den anderen Teilräumen möglichst wenig gestört werden.

An der Außenseite des Gehäuses 12 kann ein Bedienfeld 13 angeordnet sein, mit welchem insbesondere die klimatischen Bedingungen im Innenraum des Laborschranks 10, beispielsweise die Temperatur oder die Feuchte, eingestellt werden können.

Grundsätzlich ist es selbstverständlich möglich, dass der Laborschrank 10 auch zwei oder mehrere Außentüren 14 aufweisen kann.

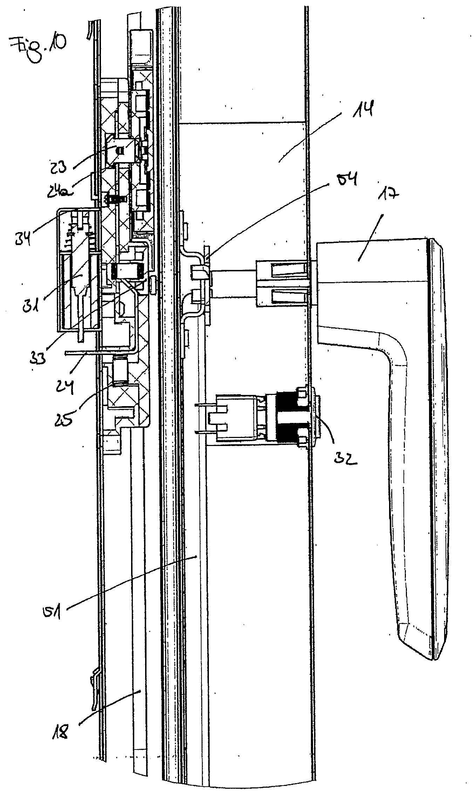

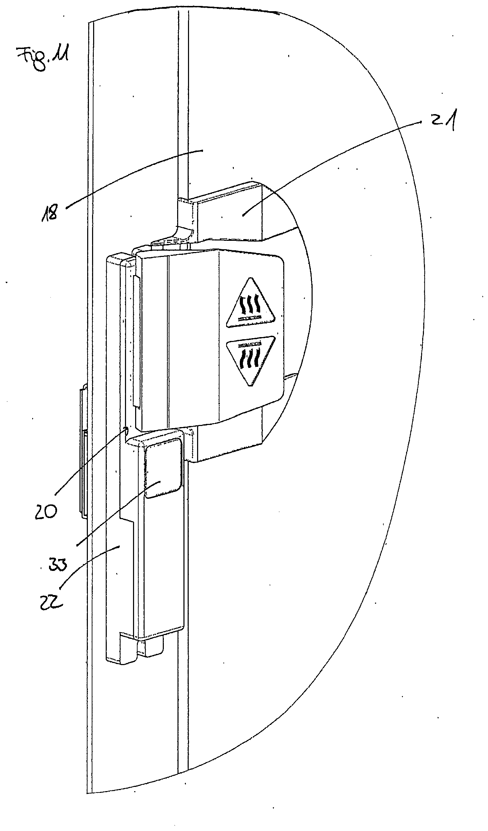

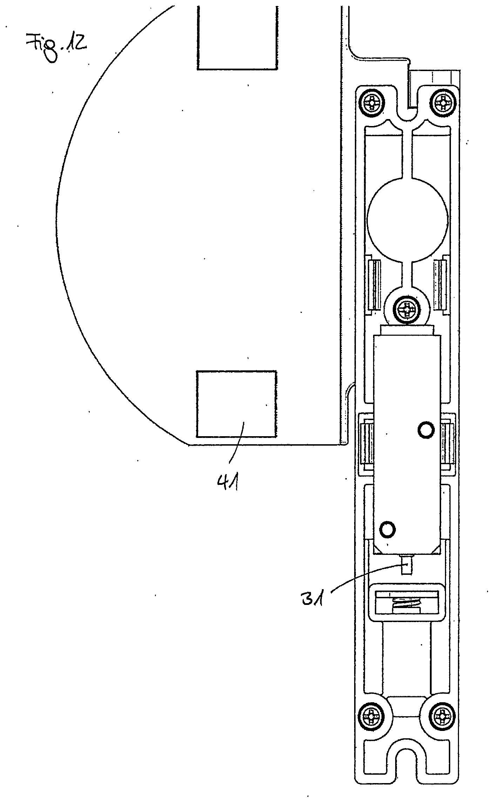

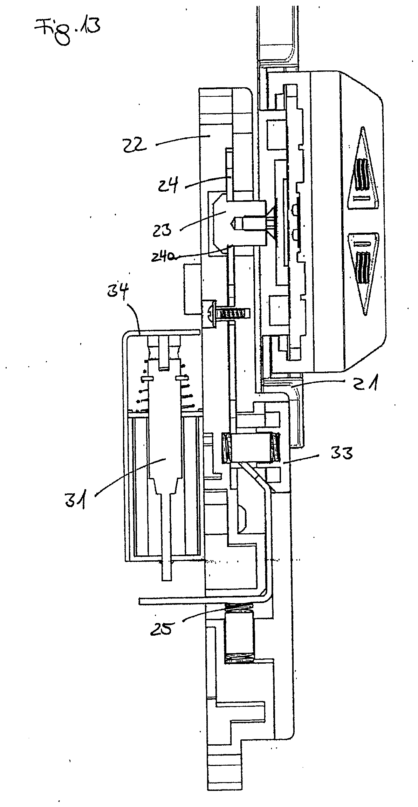

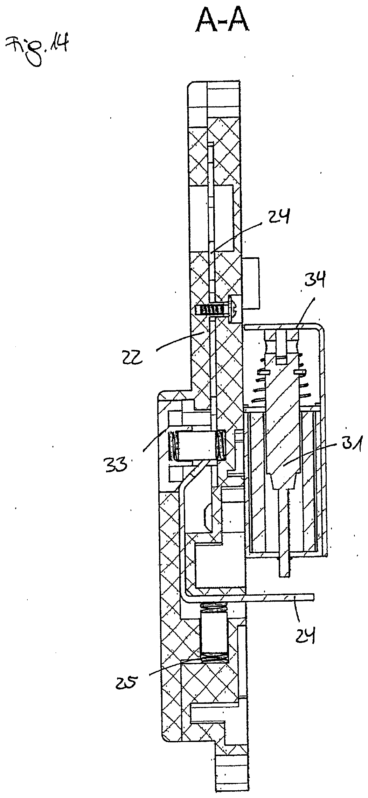

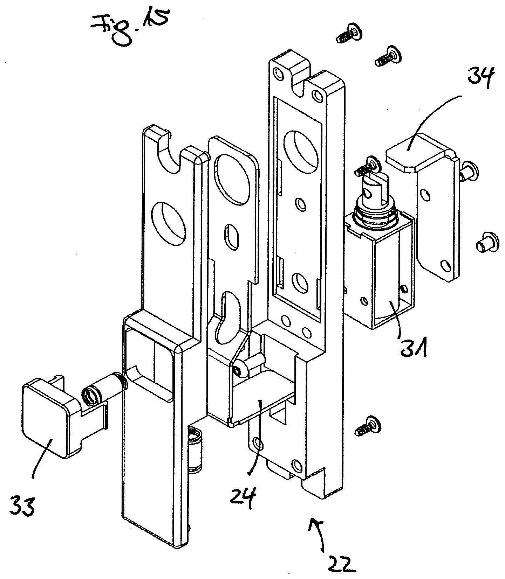

Die Figuren 10 bis 15 zeigen verschiedene Detailansichten des Verschluss- und Entriegelungsmechanismus, insbesondere des Innentürverschlusses 20 und des Außentürverschlusses 16, des in den Figuren 1 bis 3 dargestellten Laborschranks 10.

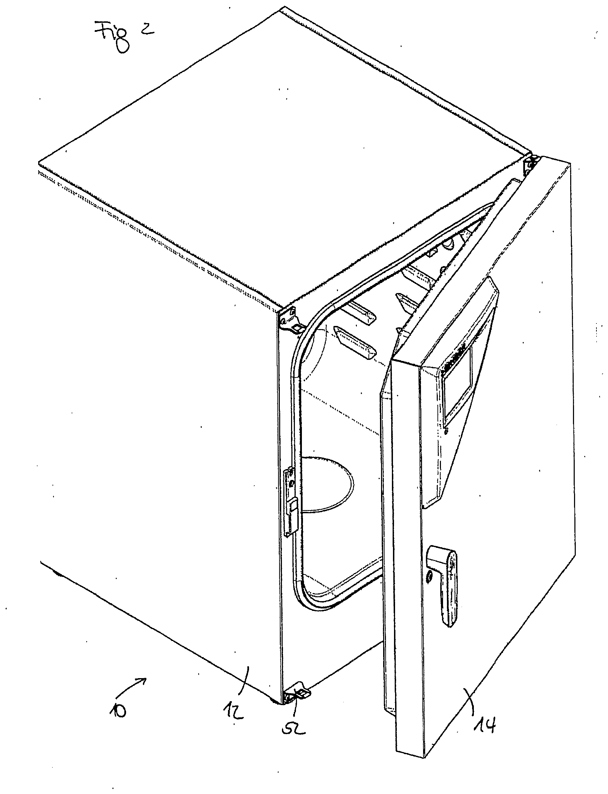

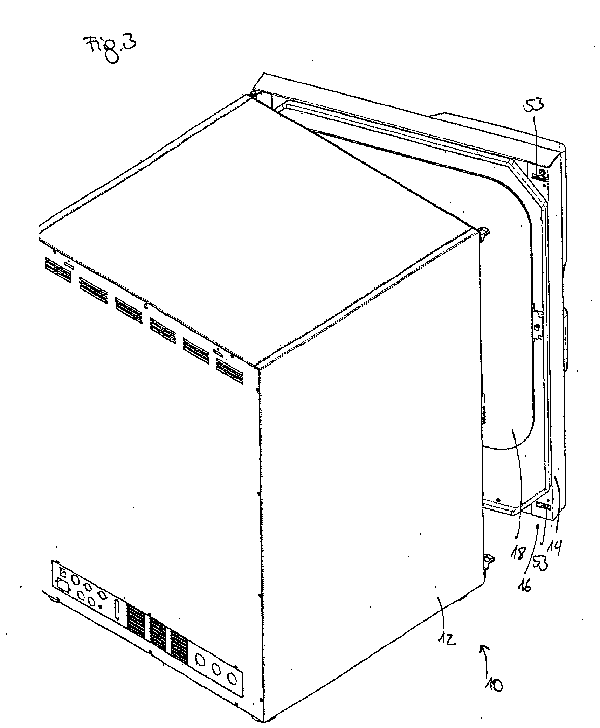

Der Außentürverschluss 16 kann beispielsweise derart ausgebildet sein, dass innerhalb der Außentür 14 zwei vertikal verlaufende an einer Exzenterscheibe 54 angelenkte Stangen, von welchen eine Stange 51 in Figur 10 erkennbar ist, bei Drehen des Handgriffs 17 in eine Richtung in vertikaler Richtung auseinander und bei Zurückdrehen des Handgriffs 17 aufeinander zu verschoben werden. Wird die Außentür 14 geschlossen, greifen zwei an den der Außentür 14 zugewandten Stirnseiten der seitenwände des Gehäuses angeordnete Laschen 52, welche insbesondere in den Figuren 1 und 2 erkennbar sind, in zwei auf der Innenseite der Außentür 14 angeordnete Schlitze 53, welche insbesondere in Figur 3 erkennbar sind, ein. Wird anschließend der Handgriff 17 derart gedreht, dass die Stangen 51 in vertikaler Richtung voneinander weggeschoben werden, greifen die zu dem Handgriff 17 beabstandeten Enden der Stangen 51 in die Laschen 52 ein und sichern auf diese Weise die Außentür 14 in der geschlossenen Position gegen unbeabsichtigtes Verschwenken. Bei Zurückdrehen des Handgriffs 17 werden die Stangen 51 wieder aufeinander zugeschoben und geben die Laschen 52 frei, so dass die Außentüre 14 wieder geöffnet werden kann.

Der Innentürverschluss 20 ist als mechanischer Verschluss ausgebildet und kann einen Rastmechanismus aufweisen. Der Innentürverschluss 20 des in den Figuren 10 bis 15 dargestellten Ausführungsbeispiels weist ein an der Innentür 18 befestigtes Innentürverschlussoberteil 21 und ein an der der Außentür 14 zugewandten Stirnseite der Seitenwand des Gehäuses 12 befestigtes Innentürverschlussunterteil 22 auf.

Der Rastmechanismus des Innentürverschlusses 20 kann einen an der Innentür 18, insbesondere dem Innentürverschlussoberteil 21, angeordneten Rastkopf 23 aufweisen, welcher mit einem mit dem Gehäuse 12, insbesondere dem Innentürverschlussunterteil 22, verbundenen Riegel 24 zusammenwirkt, insbesondere durch Eingreifen in eine Öffnung 24a des Riegels 24. Der Riegel 24 ist insbesondere durch eine Feder 25 federbelastet. Beim Schließen der Innentür 18 trifft der Rastkopf 23 auf den Riegel 24 und verschiebt diesen gegen die Kraft der Feder 25, bis der Rastkopf 23 in der Öffnung 24a des Riegels 24 einrastet.

Der Laborschrank 10 weist erste Mittel 30 zum Entriegeln des Innentürverschlusses 20 der Innentür 18 auf, welche bei geschlossener Außentür 14 betätigbar sind. Die ersten Mittel 30 sind elektrisch ansteuerbar und können beispielsweise als magnetisch erregbare Schubstange 31 ausgebildet sein. Ein Betätigungselement 32, mit welchem die Stromzufuhr eingeschaltet werden kann, ist an der Außenseite des Gehäuses 12, beispielsweise an der Außentür 14 angeordnet und kann beispielsweise in der Nähe des Handgriffs 17 der Außentür 14 angeordnet sein oder in das Bedienfeld 13 integriert sein. Wird die Stromzufuhr eingeschaltet, wird die Schubstange 31 ausgefahren und drückt gegen den Riegel 24, um diesen gegen die Kraft der Feder 25 zu verschieben und den Rastverschluss zu entriegeln, insbesondere den Rastkopf 23 aus der Öffnung 24a des Riegels 24 freizugeben. Wird die Stromzufuhr zu der Schubstange 31 wieder unterbrochen, wird die Schubstange 31 federbelastet rückgestellt, insbesondere gegen einen Anschlag 34. Möchte der Benutzer somit vor dem Öffnen der Außentür 14 auch die Innentür 18 bereits entriegeln, kann er bei geschlossener Außentür 14 die ersten Mittel 30 betätigen, um den Innentürverschluss 20 zu entriegeln.

Es kann vorgesehen sein, dass der Innentürverschluss 20 auch nach dem Öffnen der Außentür 14, wenn das Betätigungselement 32 dabei nicht gedrückt wurde, manuell zu entriegeln. Dazu ist an dem Innentürverschluss 20, insbesondere an dem Innentürverschlussunterteil 22, ein Druckknopf 33, welcher insbesondere federbelastet ist, angeordnet. Das Drücken des Druckknopfes 33 gegen die Kraft der den Druckknopf 33 belastenden Feder bewirkt ein Verschieben des Riegels 24 gegen die Federkraft der den Riegel 24 belastenden Feder 25 derart weit, dass ebenfalls der Rastkopf 23 aus der Öffnung 24a des Riegels 24 freigegeben wird und die Innentür 18 geöffnet werden kann.

Der Laborschrank 10 weist zweite Mittel 40 auf, welche derart ausgebildet sind, dass beim Öffnen der Außentür 14 die Innentür 18 ebenfalls öffnet. Bei dem in den Figuren 10 bis 15 dargestellten Ausführungsbeispielen sind die zweiten Mittel 40 als zwischen der Außentür 14 und der Innentür 18 angeordneter Mitnehmer ausgebildet, wobei der Mitnehmer insbesondere als Permanentmagnet 41 ausgebildet ist. Der Permanentmagnet 41 kann an der Innentür 18 oder der Außentür 14 angeordnet sein und ist im vorliegenden Ausführungsbeispiel an der Innentür 18, insbesondere an dem Innentürverschlussoberteil 21, angeordnet. Da der Innentürverschluss 20 bei geschlossener Außentür 14 an der Außentür 14 nahezu anliegt oder vorteilhafterweise auch in einer Mulde auf der Innenseite der Außentür 14 zu liegen kommen kann, und zudem die Innenseite der Außentür 14 aus einem magnetisierbaren Material gefertigt ist oder alternativ an der Innenseite der Außentür 14 ein magnetisierbares Element angeordnet ist, wird die Innentür 18 von der Außentür 14 magnetisch angezogen und bei Öffnen der Außentür 14 mitgenommen. Insbesondere kann die magnetische Kopplung zwischen der Innentür 18 und der Außentür 14 die Relativbewegung zwischen Innentür 18 und der Außentür 14 ausgleichen, indem der Permanentmagnet 41 über die Innenfläche der Außentür 14 gleitet.

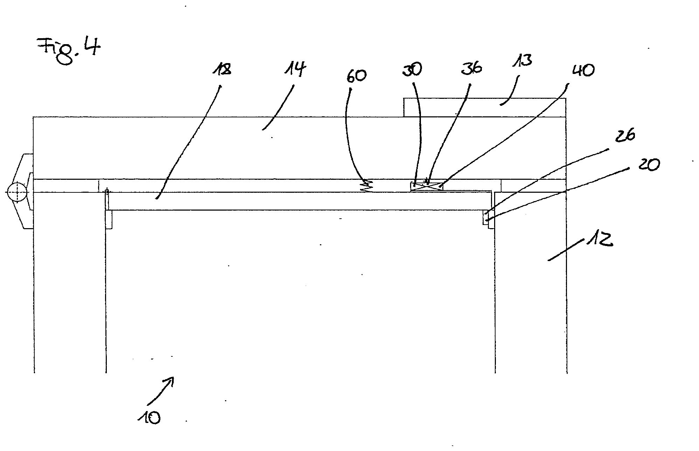

Figur 4 zeigt einen Querschnitt durch den Laborschrank 10 gemäß Figur 1 mit einem alternativen Ausführungsbeispielen des Verschluss- und Entriegelungsmechanismus. Der Innentürverschluss 20 ist in diesen Ausführungsbeispielen als mechanischer Verschluss mit einem Permanentmagneten 26 ausgebildet. Anstelle eines Permanentmagnetverschlusses kann auch eine anderer mechanischer Verschluss gewählt werden, welcher sich durch Ausüben einer Zugkraft in Öffnungsrichtung der Innentür 18 öffnen lässt, beispielsweise ein Verschluss beruhend auf einem Klemmmechanismus, einem Federmechanismus, einem Rastmechanismus, einem Schnappmechanismus und/oder einem Kugelschnappmechanismus.

Die ersten Mittel 30 sind erfindungsgemäß als Elektromagnet 36 ausgebildet, welcher entweder an der Innenseite der Außentür 14 oder der Außenseite der Innentür 18 angeordnet sein kann. Ist der Elektromagnet 36 an der Innentür 18 angeordnet, kann er im aktivierten Zustand eine magnetische Kupplung mit der Außentür 14, falls diese aus magnetisierbarem Material gefertigt ist, oder einem an der Außentür 14 angeordneten magnetisierbaren Element herstellen. Ist der Elektromagnet 36 an der Außentür 14 angeordnet, kann er entsprechend mit der Innentür 18 oder einem an der Innentür 18 angeordneten magnetisierbaren Element zusammenwirken. Der Elektromagnet 36 kann über ein Betätigungselement, welches an der Außenseite des Gehäuses 12, beispielsweise an der Außentür 14 als separates Element oder integriert in das Bedienfeld 13 angeordnet ist, aktiviert werden. Die Haltekraft des Elektromagneten 36 ist dabei größer als die Haltekraft des Permanentmagnetverschlusses 26, so dass beim Öffnen der Außentür 14 auch der Innentürverschluss 20 entriegelt und geöffnet wird. Die derart als Elektromagnet 36 ausgebildeten ersten Mittel 30 bilden dabei gleichzeitig auch die zweiten Mittel 40, welche bewirken, dass beim Öffnen der Außentür 14 die Innentür 18 ebenfalls öffnet. Die Relativbewegung zwischen der Außentür 14 und der Innentür 18 beim Schwenken der Türen, welche bedingt ist durch die unterschiedlichen Drehpunkte der beiden Türen 14, 18, kann dadurch ausgeglichen werden, dass der Elektromagnet 36 über die glatte Türfläche gleiten kann. Ist der Elektromagnet 36 an der Außentür 14 angeordnet, kann ein eventuell erforderliches Kabel vom Betätigungselement zum Elektromagnet 36 vollständig innerhalb der Außentür 14 verlegt werden und muss nicht um den Drehpunkt der Innentür 18 verlegt werden. Zwischen der Außentür 14 und der Innentür 18 ist vorzugsweise an der Außentür 14 ein Dämpfungselement 60, beispielsweise ein Federelement oder ein elastisches Element wie beispielsweise ein Gummidämpfer, angeordnet, welches bei Schließen der Außentür 14 an der Innentür 18 zur Anlage kommt und die Innentür 18 derart weit schließt, dass der Innentürverschluss 20 wieder verriegelt.

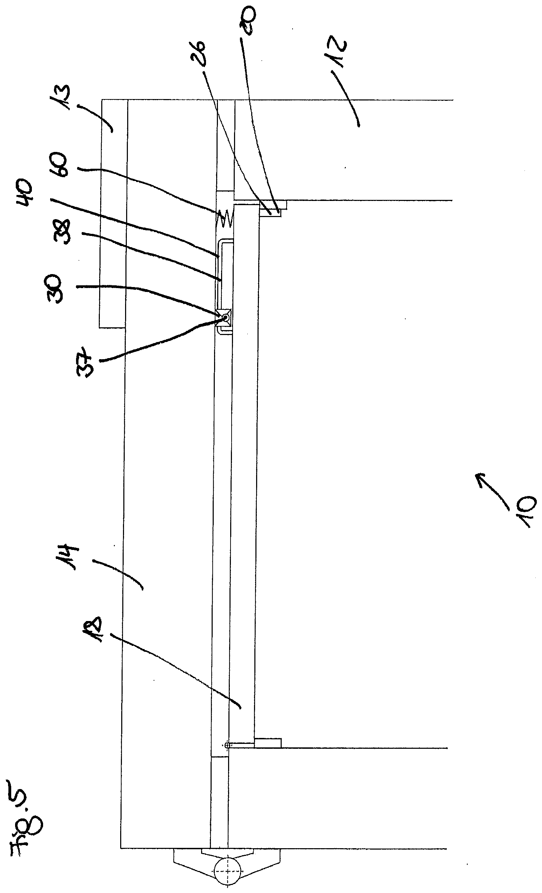

Das in Figur 5 dargestellte Ausführungsbeispiel des Laborschranks 10 unterscheidet sich von dem in Figur 4 dargestellten Laborschrank 10 lediglich durch die Ausgestaltung der ersten Mittel 30 und der zweiten Mittel 40. Die ersten Mittel 30 sind als an einer von Außentür 14 und Innentür 18, vorliegend insbesondere an der Außentür 14, angeordnete elektromagnetisch erregbare Schubstange 37 ausgebildet. Die Bewegungsrichtung der Schubstange 37 verläuft dabei insbesondere parallel zur Schwenkachse der Außentür 14 oder der Innentür 18. Im ausgefahrenen Zustand hintergreift die Schubstange 37 ein an dem anderen von Außentür 14 und Innentür 18, vorliegend der Innentür 18, angeordnetes Element 38, wobei das Element 38 griffartig oder bügelartig ausgebildet ist und an beiden Enden einen Anschlag für die Schubstange 37 bildet. Bei Relativbewegung zwischen der Außentür 14 und der Innentür 18 beim Öffnen der beiden Türen, 14, 18 kann die Schubstange 37 somit über eine gewisse Wegstrecke an dem Element 38 entlang gleiten, ist jedoch in dem Weg beschränkt. Wird der Öffnungswinkel der Außentür 14 erreicht, an dem die Schubstange 37 an einem Ende des Elements 38 anschlägt, kann die Außentür 14 nur dann weiter geöffnet werden, wenn die Schubstange 37 wieder eingefahren wird. Hintergreift die Schubstange 37 das Element 38, wird dadurch eine Kopplung zwischen der Außentür 14 und der Innentür 18 erreicht, die einerseits beim Öffnen der Außentür 14 den Innentürverschluss 20 entriegelt, andererseits gleichzeitig aber auch die zweiten Mittel 40 bilden, welche bewirken, dass die Innentür 18 beim Öffnen der Außentür 14 ebenfalls öffnet.

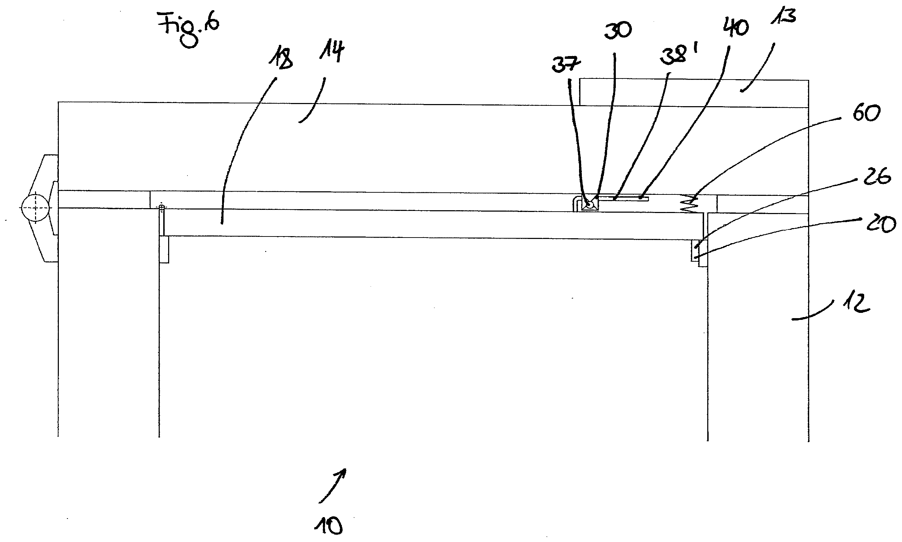

Das in Figur 6 dargestellte Ausführungsbeispiel des Laborschranks 10 unterscheidet sich von dem in Figur 5 dargestellten Laborschrank 10 lediglich durch die Ausgestaltung der ersten Mittel 30 und der zweiten Mittel 40. Statt des Elements 38, welches bei dem in Figur 5 dargestellten Ausführungsbeispiel des Laborschranks 10 bügelartig mit zwei Befestigungspunkten ausgebildet ist, wird bei dem in Figur 6 dargestellten Ausführungsbeispielen des Laborschranks 10 ein Element 38' verwendet, welches lediglich an einer Seite an der Innentür 18 befestigt ist und zum anderen Ende hin offen ist. Die derart ausgebildeten ersten Mittel 30 entriegeln den Innentürverschluss 20, weil der Permanentmagnetverschluss 26 gelöst wird, wenn die Schubstange 37 ausgefahren wird und das Element 38' hintergreift, da beim Öffnen der Außentür 14 der Innentürverschluss 20 geöffnet wird. Gleichzeitig bilden die derart ausgebildeten ersten Mittel 30 auch die zweiten Mittel 40, da die Innentür 18 ebenfalls öffnet, wenn die Außentür 14 geöffnet wird. Beim Öffnen der Außentür 14 kann die Schubstange 37 über einen gewissen Öffnungswinkel entlang des Elements 38' gleiten, wobei die Innentür 18 weiter mitgenommen wird. Wird ein bestimmter Öffnungswinkel erreicht, gleitet die Schubstange 37 an dem freien Ende des Elements 38' aus der Mitnahmeposition heraus und gibt die Innentür 18 frei.

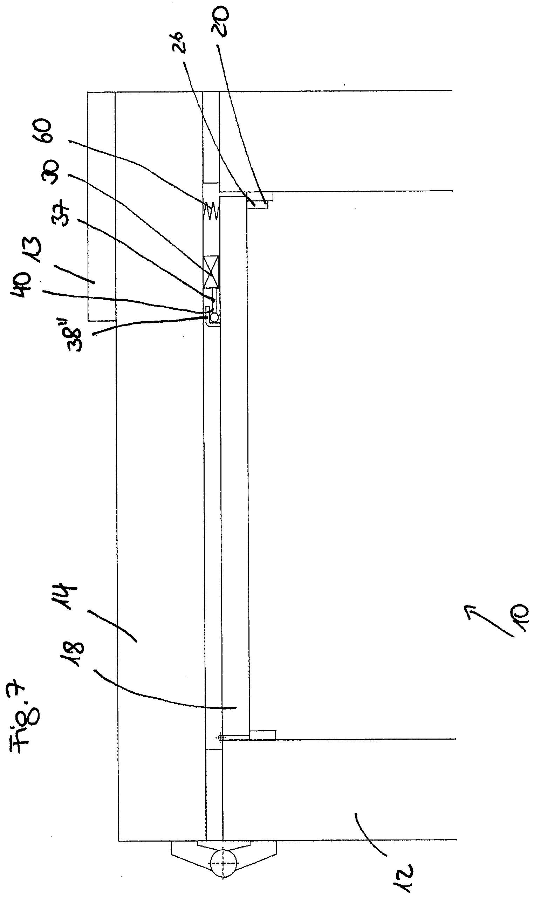

Das in Figur 7 dargestellte Ausführungsbeispiel des Laborschranks 10 unterscheidet sich von dem in Figur 6 dargestellten Ausführungsbeispiel des Laborschranks 10 lediglich durch die Ausgestaltung der ersten Mittel 30 und der zweiten Mittel 40. Die magnetisch erregbare Schubstange 37 ist bei dem in Figur 7 dargestellten Ausführungsbeispiel des Laborschranks 10 derart angeordnet, dass die Bewegungsrichtung der Schubstange 37 senkrecht zur Drehachse der Außentür 14 oder der Innentür 18 und insbesondere parallel zur Fläche der Innentür 18 oder der Außentür 14 verläuft. Die Schubstange 37 kann im ausgefahrenen Zustand nach Aktivierung der Stromzufuhr von außerhalb des Gehäuses 12 in ein einseitig offenes Element 38" eingreifen und auf diese Weise sowohl die erstem Mittel 30 zum Entriegeln des Innentürverschlusses 20 als auch die zweiten Mittel 40 zur Mitnahme der Innentür 18 bei Öffnen der Außentür 14 bilden. Aufgrund der Relativbewegung zwischen der Außentür 14 und der Innentür 18 beim Öffnen der Außentür 14 kann bei einem bestimmten Öffnungswinkel die Schubstange 37 außer Eingriff mit dem Element 38" gelangen und die Innentür 18 wieder frei geben.

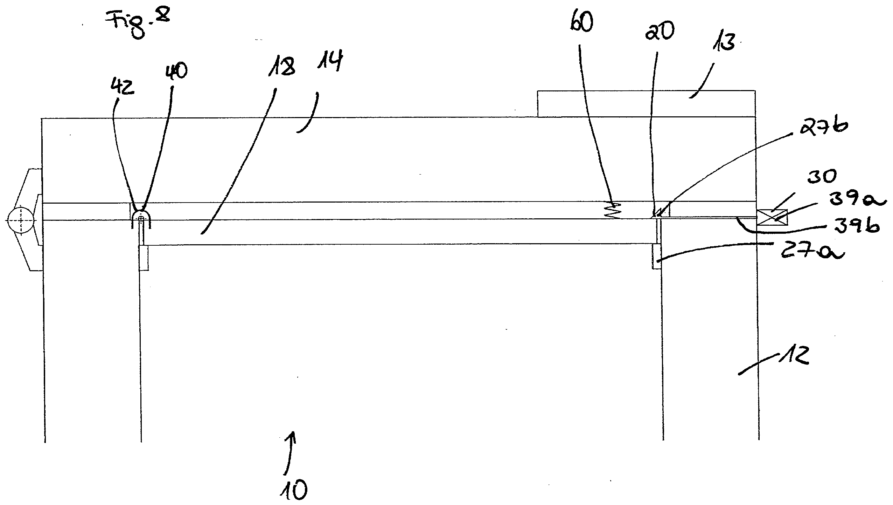

Figur 8 zeigt ein weiteres Ausführungsbeispiel des Laborschranks 10, welches sich von dem in Figur 7 dargestellten Ausführungsbeispiel des Laborschranks 10 durch die Ausgestaltung des Innentürverschlusses 20 sowie die Ausgestaltung der ersten Mittel 30 und der zweiten Mittel 40 unterscheidet.

Der Innentürverschluss 20 ist als mechanischer Verschluss ausgebildet, welcher einen Schnappverschluss mit einem federbelasteten Vorsprung 27b aufweist. Die Innentür 18 liegt in der geschlossenen Position an einem Anschlag 27a an und wird durch den federbelasteten Vorsprung 27b, welcher insbesondere am Gehäuse 12 angeordnet ist, in dieser Position verriegelt. Beim Zudrücken der Innentüre 18 wird der Vorsprung 27b gegen die Kraft der Feder verschoben, so dass die Innentür 18 hinter dem Vorsprung 27b einschnappen kann.

Die ersten Mittel 30 zum Entriegeln des Innentürverschlusses 20 sind als Elektromagnet 39a ausgebildet, welcher insbesondere auf der Außenseite des Gehäuses 12 angeordnet sein kann. Der Elektromagnet 39a ist über ein Verbindungselement 39b, welches beispielsweise als Stab oder Blech ausgebildet sein kann, mit dem Vorsprung 27b verbunden. Vorzugsweise ist das Verbindungselement 39b durch den Dichtungsspalt zwischen der Außentür 14 und der Stirnseite einer Seitenwand des Gehäuses 12 nach innen zu dem Vorsprung 27b geführt. Der Elektromagnet 39a kann bei geschlossener Außentür 14 aktiviert, insbesondere bestromt werden, beispielsweise durch Betätigen des Betätigungselements 32, wodurch mittels des Verbindungselements 39b der Vorsprung 27b derart verschoben, insbesondere zurückgezogen, wird, dass die Innentür 18 freigegeben wird. Nach Freigabe der Innentür 18 wird der Vorsprung 27b federbelastet in seine Ausgangsposition geführt.

Die zweiten Mittel 40 sind bei diesem Ausführungsbeispiel als die Innentür 18 beaufschlagende Feder, beispielsweise als Torsionsfeder 42, ausgebildet. Sobald die Außentür 14 geöffnet und der Innentürverschluss 20 entriegelt ist, wird die Innentür 18 durch die Torsionsfeder 22 belastet in eine geöffnete Position gedrückt.

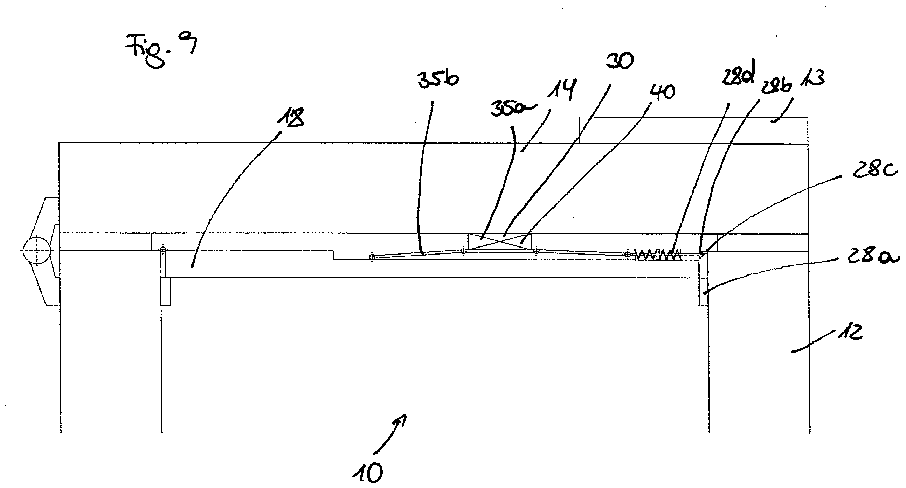

Das in Figuren 9 dargestellte Ausführungsbeispiel des Laborschranks 10 unterscheidet sich von dem in Figur 8 dargestellten Ausführungsbeispiel durch die Ausgestaltung des Innentürverschlusses 20 sowie durch die Ausgestaltung der ersten Mittel 30 und der zweiten Mittel 40.

Der Innentürverschluss 20 ist als mechanischer Verschluss ausgebildet, welcher einen Schnappverschluss aufweist. Der Schnappverschluss weist einen durch eine Feder 28d federbelasteten Vorsprung 28b auf, welcher an der Innentür 18 angeordnet ist. In der geschlossenen Position schlägt die Innentür 18 an einem Vorsprung 28c an, welcher an der Innenseite der Seitenwand des Gehäuses 12 angeordnet ist. In Abstand zu dem Anschlag 28a ist ein Vorsprung 28c angeordnet, welcher beim Zudrücken der Innentür 18 den federbelasteten Vorsprung 28b gegen die Kraft der Feder 28d zurückdrückt, wobei schließlich in der geschlossenen Position der federbelastete Vorsprung 28b hinter dem Vorsprung 28c einschnappt.

Die ersten Mittel 30 zum Entriegeln des Innentürverschlusses 20 weisen einen Elektromagneten 35a auf, welcher an der Innenseite der Außentür 14 angeordnet ist. Das Aktivieren des Elektromagneten 35a kann bei geschlossener Außentür 14 erfolgen, beispielsweise durch Betätigen eines Betätigungsknopfs, welcher an der Außenseite des Gehäuses 12, beispielsweise an der Außenseite der Außentür 14, angeordnet ist oder auch in das Bedienfeld 13 integriert sein kann. Im aktivierten Zustand zieht der Elektromagnet 35a den federbelasteten Vorsprung 28b gegen die Kraft der Feder 28d, zurück, insbesondere dadurch, dass er an einem Hebelmechanismus, beispielsweise einem Hebelgestänge 35b, angreift, welches an dem federbelasteten Vorsprung 28b angeordnet ist. Das Hebelgestänge 35b weist wenigstens zwei Gelenke auf, wodurch ein Auslenken eines Teils des Hebelgestänges 35b quer zur Ebene der Innentür 18 bewirkt durch die Anziehungskraft des Elektromagneten 35a erfolgen kann, was in einer Bewegung des federbelasteten Vorsprungs 28b parallel zur Ebene der Innentür 18 resultiert. Durch Aktivieren des Elektromagneten 35a wird dadurch der Innentürverschluss 20 entriegelt.

Die ersten Mittel 30 bilden aber gleichzeitig auch die zweiten Mittel 40, welche derart ausgebildet sind, dass beim Öffnen der Außentür 14 die Innentür 18 ebenfalls öffnet, da der aktivierte Elektromagnet 35a eine magnetische Kopplung zu dem Hebelgestänge 35b bewirkt und dadurch ein Mitnehmer bereitgestellt wird, welcher bewirkt, dass die Innentür 18 beim Öffnen der Außentür 14 mit aufgezogen wird. Durch die Gelenke des Hebelgestänges 35b kann auch eine Relativbewegung zwischen der Außentür 14 und der Innentür 18 beim Öffnen der Türen 14, 18 ausgeglichen werden.

Bei sämtlichen beschriebenen Ausführungsbeispielen ist vorzugsweise ein manuelles Öffnen des Innentürverschlusses 20 möglich, falls die Außentür 14 geöffnet wurde, ohne den Innentürverschluss 20 zu entriegeln.

### Bezugszeichenliste

- 10: Laborschrank
- 13: Bedienfeld
- 12: Gehäuse
- 14: Außentür
- 16: Außentürverschluss
- 17: Handgriff
- 18: Innentür
- 20: Innentürverschluss
- 21: Innentürverschlussoberteil
- 22: Innentürverschlussunterteil
- 23: Rastkopf
- 24: Riegel
- 24a: Öffnung
- 25: Feder
- 26: Permanentmagnet
- 27a: Anschlag
- 27b: Vorsprung
- 28a: Anschlag
- 28b: federbelasteter Vorsprung
- 28c: Vorsprung
- 28d: Feder
- 30: erste Mittel
- 31: Schubstange
- 32: Betätigungselement
- 33: Druckknopf
- 34: Anschlag
- 35a: Elektromagnet
- 35b: Hebelgestänge
- 36: Elektromagnet
- 37: Schubstange
- 38: Element
- 38': Element
- 38": Element
- 39a: Elektromagnet
- 39b: Verbindungselement
- 40: zweite Mittel
- 41: Permanentmagnet
- 42: Torsionsfeder
- 51: Stange
- 52: Lasche
- 53: Schlitz
- 54: Exzenterscheibe
- 60: Dämpfungselement

## Patentansprüche

1. Laborschrank (10), beispielsweise Kälteschrank, Wärmeschrank, Trockenschrank oder Brutschrank, mit einem wenigstens eine Außentür (14) mit einem Außentürverschluss (16) aufweisenden Gehäuse (12), welches wenigstens einen Innenraum aufweist, der durch eine Innentür (18) mit einem Innentürverschluss (20) verschließbar ist, wobei der Innentürverschluss (20) ein mechanischer Verschluss ist, dass der Laborschrank (10) bei geschlossener Außentür (14) betätigbare erste Mittel (30) zum Entriegeln des Innentürverschlusses (20) der Innentür (14) aufweist, welche elektrisch ansteuerbar sind, und dass der Laborschrank (10) zweite Mittel (40) aufweist, welche derart ausgebildet sind, dass beim Öffnen der Außentür (14) die Innentür (18) ebenfalls öffnet, **dadurch gekennzeichnet, dass** die ersten Mittel (30) als an einer von Außentür (14) und Innentür (18) angeordneter Elektromagnet (36, 39a, 35a) ausgebildet ist, welcher im aktivierten Zustand mit dem anderen von Außentür (14) und Innentür (18) oder mit einem an dem anderen von Außentür (14) und Innentür (18) angeordneten magnetisierbaren Element zusammenwirkt und dessen Haltekraft größer ist als die Verschlusskraft des mechanischen Verschlusses.

2. Laborschrank nach einem der vorhergehenden Ansprüche, **dadurch gekennzeichnet, dass** der mechanische Verschluss einen Permanentmagneten (26), einen Klemmmechanismus, einen Rastmechanismus, einen Federmechanismus, einen Schnappmechanismus und/oder einen Kugelschnappmechanismus aufweist.

3. Laborschrank nach einem der vorhergehenden Ansprüche,
**dadurch gekennzeichnet, dass** die ersten Mittel (30) als insbesondere an einer von Außentür (14) und Innentür (18) angeordneter Elektromagnet (39a, 35a) ausgebildet sind, welcher im aktivierten Zustand den mechanischen Verschluss entriegelt.

4. Laborschrank nach Anspruch 4,
**dadurch gekennzeichnet, dass** der Elektromagnet (39a, 35a) im aktivierten Zustand den mechanischen Verschluss durch Bewegen eines Elements des mechanischen Verschlusses, beispielsweise durch Verschieben eines Riegels (24) und/oder Verschieben oder Freigeben eines Rastelements, entriegelt.

5. Laborschrank nach Anspruch 3 oder 4,
**dadurch gekennzeichnet, dass** der mechanische Verschluss einen Schnappverschluss mit einem federbelasteten Vorsprung (28b) aufweist, und der Elektromagnet (35a) im aktivierten Zustand den Vorsprung (28b) insbesondere über einen Hebelmechanismus, beispielsweise ein Hebelgestänge (35b), gegen die Federkraft zurückzieht, um den Schnappverschluss zu entriegeln.

6. Laborschrank nach einem der vorhergehenden Ansprüche,
**dadurch gekennzeichnet, dass** die ersten Mittel (30) als magnetisch erregbare Schubstange (31) ausgebildet sind und der mechanische Verschluss einen Rastmechanismus mit einem federbelasteten Riegel (24) mit einer Öffnung (24a) aufweist, in welche ein Rastkopf (23) eingreift, und die Schubstange (31) im aktivierten Zustand den Riegel (24) gegen die Federkraft bewegt, um den Rastverschluss zu entriegeln.

7. Laborschrank nach einem der vorhergehenden Ansprüche,
**dadurch gekennzeichnet, dass** die ersten Mittel (30) als an einer von Außentür (14) und Innentür (18) angeordnete elektromagnetisch erregbare Schubstange (37) ausgebildet sind, welche im ausgefahrenen Zustand ein an dem anderen von Außentür (14) und Innentür (18) angeordnetes Element (38, 38', 38") hintergreift.

8. Laborschrank nach einem der vorhergehenden Ansprüche,
**dadurch gekennzeichnet, dass** die zweiten Mittel (40) als zwischen der Außentür (14) und der Innentür (18) angeordneter Mitnehmer ausgebildet sind.

9. Laborschrank nach Anspruch 8,
**dadurch gekennzeichnet, dass** der Mitnehmer als an einer von Außentür (14) und Innentür (18) angeordneter Permanentmagnet (41) oder Elektromagnet (35a) ausgebildet ist, welcher mit dem anderen von Außentür (14) und Innentür (18) oder mit einem an dem anderen von Außentür (14) und Innentür (18) angeordnetem magnetisierbaren Element zusammenwirkt.

10. Laborschrank nach Anspruch 8 oder 9,
**dadurch gekennzeichnet, dass** der Mitnehmer als an einer von Außentür (14) und Innentür (18) angeordneter elektrisch ausfahrbarer Stößel oder Riegel oder elektromagnetisch erregbare Schubstange (37) ausgebildet ist, welcher oder welche im ausgefahrenen Zustand ein an dem anderen von Außentür (14) und Innentür (18( angeordnetes Element (38, 38', 38") hintergreift.

11. Laborschrank nach einem der vorhergehenden Ansprüche,
**dadurch gekennzeichnet, dass** die zweiten Mittel (40) als die Innentür (18) beaufschlagende Feder, beispielsweise als Torsionsfeder (42), ausgebildet sind.

12. Laborschrank nach einem der vorhergehenden Ansprüche,
**dadurch gekennzeichnet, dass** die ersten Mittel (30) gleichzeitig die zweiten Mittel (40) bilden.

13. Laborschrank nach einem der vorhergehenden Ansprüche,
**dadurch gekennzeichnet, dass** der Innentürverschluss)20) bei geöffneter Außentür (14) manuell entriegelbar ist.

14. Laborschrank nach einem der vorhergehenden Ansprüche,
**dadurch gekennzeichnet, dass** an der Außentür (14) oder der Innentür (18) ein Dämpfungselement (60), beispielsweise ein Federelement oder ein elastisches Element, angeordnet ist, welches bei einer Schließbewegung der Außentür (14) zwischen der Außentür (14) und der Innentür (18) zur Anlage kommt.

15. Laborschrank nach einem der vorhergehenden Ansprüche,
**dadurch gekennzeichnet, dass** die Innentür (18) zumindest abschnittsweise aus transparentem Material, insbesondere Glas, gefertigt ist.

16. Laborschrank nach einem der vorhergehenden Ansprüche,
**dadurch gekennzeichnet, dass** der Laborschrank (10) mehrere Innentüren (18) aufweist und dass die ersten Mittel (30) derart ausgebildet sind, dass durch die ersten Mittel (30) eine oder mehrere der Innentüren (18) entriegelbar sind.

## Claims

1. Laboratory cabinet (10), for example a cooling cabinet, warming cabinet, drying cabinet or an incubation cabinet, with at least one outer door (14) with a housing comprising an outer door locking device (16) and having at least one inner chamber that can be closed with an inner door (18) that has an inner door locking device (20),
wherein the inner door locking device (20) is a mechanical lock and in that the laboratory cabinet (10) comprises first means (30) that can be operated to unlock the inner door locking device (20) when the outer door (14) is closed and which are implemented such that, when the outer door (14) is opened, the inner door (18) also opens,
**characterized in that** the first means (30) are implemented as an electromagnet (36, 39a, 35a) that is arranged on the outer door (14) and on the inner door (18) and which interacts, when in an activated state, with the other magnetisable element of the outer door (14) and the inner door (18) or with a different element arranged on the magnetisable element of the outer door (14) and the inner door (18) and the holding force of which is greater than the locking force of the mechanical lock.

2. Laboratory cabinet in accordance with the preceding claim,
**characterized in that** the mechanical lock comprises a permanent magnet (26), a clamping mechanism, a latching mechanism, a spring mechanism, a snap mechanism, or a ball-catch mechanism.

3. Laboratory cabinet in accordance with any of the preceding claims,
**characterized in that** the first means (30) are implemented, in particular, on an electromagnet (39a, 35a) arranged on the outer door (14) and inner door (18) and which, when in an activated state, unlocks the mechanical lock.

4. Laboratory cabinet in accordance with claim 4,
**characterized in that**, in an activated state, the electromagnet (39a, 35a) unlocks the mechanical lock by moving an element of the mechanical lock, for example, by moving shifting a latch (24) and/or by shifting or releasing a snap element.

5. Laboratory cabinet in accordance with claim 3 or 4,
**characterized in that** the mechanical lock comprises a snap lock with a spring-biased tab (28b) and, when in an activated state, the electromagnet (35a) pulls the tab (28b) back, in particular over a lever mechanism, for example, a lever linkage (35b) against the force of the spring in order to unlock the snap lock.

6. Laboratory cabinet in accordance with any of the preceding claims,
**characterized in that** the first means (30) are implemented as magnetically excitable push rod (31) and **in that** the mechanical lock comprises a latching mechanism with a spring-biased latch (24) with an opening (24a) in which a latching head (23) engages, and **in that** the push rod (31), when in an active state, moves against the spring force in order to unlock the snap lock.

7. Laboratory cabinet in accordance with any of the preceding claims,
**characterized in that** the first means (30) are implemented as an electromagnetic push rod (37) arranged on the outer door (14) and on the inner door (18) and which, when in an extended state, engages with the other element (38, 38', 38") arranged on the outer door (14) and the inner door (18).

8. Laboratory cabinet in accordance with any of the preceding claims,
**characterized in that** the second means (40) are implemented as a driving element arranged between the outer door (14) and the inner door (18).

9. Laboratory cabinet in accordance with claim 8,
**characterized in that** the driving element is implemented as a permanent magnet (41) or as an electromagnet (35a) arranged on the outer door (14) and inner door (18) which interacts with the other magnetisable element arranged on the outer door (14) and the inner door (18) or with an element arranged on the other magnetisable element arranged on the outer door (14) and the inner door (18).

10. Laboratory cabinet in accordance with claim 8 or 9,
**characterized in that** the driving element is implemented as an electrically extendable tappet or bolt or as an electromagnetically excitable push rod (37) arranged on the outer door (14) and the inner door (18) and which, in an extended state, engages with the other element (38, 38', 38") arranged on the outer door (14) and the inner door (18).

11. Laboratory cabinet in accordance with any of the preceding claims,
**characterized in that** the second means (40) are implemented as torsion springs (42) that bias the inner door (18).

12. Laboratory cabinet in accordance with any of the preceding claims,
**characterized in that** the first means (30) simultaneously form the second means (40).

13. Laboratory cabinet in accordance with any of the preceding claims,
**characterized in that** the inner door locking device (20) can be manually unlocked when the outer door (14) is open.

14. Laboratory cabinet in accordance with any of the preceding claims,
**characterized in that** a damping element (60), for example, a spring element or an elastic element, is arranged on the outer door (14) and the inner door (18) which comes to bear between the outer door (14) and the inner door (18) when a closing movement of the outer door (14) is carried out.

15. Laboratory cabinet in accordance with any of the preceding claims,
**characterized in that** the inner door (18) is at least partially made of a transparent material, in particular, of glass.

16. Laboratory cabinet in accordance with any of the preceding claims,
**characterized in that** the laboratory cabinet (10) comprises numerous inner doors (18) and **in that** the first means (30) are configured such that one or more of the inner doors (18) can be unlocked by the first means (30).

## Revendications

1. Armoire de laboratoire (10) par exemple armoire frigorifique, armoire chauffante, armoire de séchage ou incubateur comprenant au moins une porte extérieure (14) avec un boîtier (12) muni d'une serrure de porte extérieure (16), ce boîtier ayant au moins un volume intérieur qui peut se fermer avec une porte intérieure (18) munie d'une serrure de porte intérieure (20),
dans laquelle
la serrure de porte intérieure (20) est une serrure mécanique, de façon que l'armoire de laboratoire (10), lorsque la porte extérieure (14) est fermée, comporte des premiers moyens (30) commandées électriquement qui peuvent être actionnés pour déverrouiller la serrure (20) de la porte intérieure (14), et
- l'armoire de laboratoire (10) comporte des seconds moyens (40) réalisés de façon qu'en ouvrant la porte extérieure (14) la porte intérieure (18) s'ouvre également,
armoire de laboratoire **caractérisée en ce que**
les premiers moyens (30) sont réalisés sous la forme d'un électro-aimant (36, 39a, 35a) prévu sur la porte extérieure (14) et la porte intérieure (18), qui à l'état activé, coopèrent avec l'autre élément aimanté de la porte extérieure (14) et de la porte intérieure (18) ou avec un élément aimanté prévu sur la porte extérieure (14) et la porte intérieure (18) et dont la force de retenue est supérieure à la force de fermeture de la serrure mécanique.

2. Armoire de laboratoire (10) selon la revendication précédente,
**caractérisée en ce que**
la fermeture mécanique comprend un aimant permanent (26), un mécanisme de serrage, un mécanisme par accrochage, un mécanisme à ressort, un mécanisme d'enclipsage et/ou un mécanisme d'enclipsage à bille.

3. Armoire de laboratoire (10) selon l'une des revendications précédentes,
**caractérisée en ce que**
les premiers moyens (30) sont réalisés notamment sous la forme d'un électro-aimant (39a, 35a) prévus sur la porte extérieure (14) et la porte intérieure (18) et qui à l'état activé déverrouille la serrure mécanique.

4. Armoire de laboratoire (10) selon la revendication 3,
**caractérisée en ce que**
l'électro-aimant (39a, 35a), à l'état activé, déverrouille la serrure mécanique en déplaçant un élément de la serrure mécanique, par exemple en coulissant un verrou (24) et/ou en coulissant ou en libérant un élément d'accrochage.

5. Armoire de laboratoire (10) selon la revendication 3 ou 4,
**caractérisée en ce que**
la serrure mécanique comporte une serrure par enclipsage munie d'un élément en saillie (28b), chargé par un ressort et l'électro-aimant (35a), à l'état activé, tire en arrière l'élément en saillie (28b) notamment par un mécanisme de levier par exemple une tringlerie à levier (35b) contre la force du ressort pour déverrouiller la liaison par enclipsage.

6. Armoire de laboratoire (10) selon l'une des revendications précédentes,
**caractérisée en ce que**
les premiers moyens (30) sont réalisés sous la forme d'une tige-poussoir (31) à excitation magnétique et la serrure mécanique comporte un mécanisme d'accrochage avec un verrou à ressort (24) muni d'une ouverture (24a) dans laquelle vient une tête d'accrochage (23) et à l'état activé, la tige-poussoir (31) déplace le verrou (24) contre la force du ressort pour déverrouiller la liaison par accrochage.

7. Armoire de laboratoire (10) selon l'une des revendications précédentes,
**caractérisée en ce que**
les premiers moyens (30) sont réalisés sous la forme d'une tige-poussoir (37) prévue sur la porte extérieure (14) ou la porte intérieure (18) et qui à l'état déployé vient prendre derrière un élément (38, 38', 38") prévu sur l'autre porte extérieure (14) ou porte intérieure (18).

8. Armoire de laboratoire (10) selon l'une des revendications précédentes,
**caractérisée en ce que**
les seconds moyens (40) sont réalisés sous la forme d'un organe d'entraînement entre la porte extérieure (14) et la porte intérieure (18).

9. Armoire de laboratoire (10) selon la revendication 8,
**caractérisée en ce que**
l'organe d'entraînement est réalisé sous la forme d'un aimant permanent (41) ou d'un électro-aimant (35a) prévu sur la porte extérieure (14) et la porte intérieure (18), cet élément coopérant avec l'autre porte extérieure (14) ou intérieure (18) ou avec un élément magnétique prévu sur l'autre porte extérieure (14) ou intérieure (18).

10. Armoire de laboratoire (10) selon la revendication 8 ou 9,
**caractérisée en ce que**
l'organe d'entraînement est un poussoir ou un verrou ou une tige-poussoir (37) à excitation électromagnétique prévu sur la porte extérieure (14) ou la porte intérieure (18) et qui, à l'état déployé, vient prendre derrière un élément (38, 38', 38") prévu sur l'autre porte extérieure (14) ou intérieure (18).

11. Armoire de laboratoire (10) selon l'une des revendications précédentes,
**caractérisée en ce que**
les seconds moyens (40) sont réalisés sous la forme d'un ressort sollicitant la porte intérieure (18), par exemple un ressort de torsion (42).

12. Armoire de laboratoire (10) selon l'une des revendications précédentes,
**caractérisée en ce que**
les premiers moyens (30) forment en même temps les seconds moyens (40).

13. Armoire de laboratoire (10) selon l'une des revendications précédentes,
**caractérisée en ce que**
la serrure de la porte intérieure (20) se déverrouille manuellement lorsque la porte extérieure (14) est ouverte.

14. Armoire de laboratoire (10) selon l'une des revendications précédentes,
**caractérisée en ce que**
un élément amortisseur (60), par exemple un élément de ressort ou un élément élastique, est prévu sur la porte extérieure (14) ou la porte intérieure (18) et il vient en appui entre la porte extérieure (14) et la porte intérieure (18) lors du mouvement de fermeture de la porte extérieure (14).

15. Armoire de laboratoire (10) selon l'une des revendications précédentes,
**caractérisée en ce que**
la porte intérieure (18) est réalisée au moins en partie en une matière transparente, notamment en verre.

16. Armoire de laboratoire (10) selon l'une des revendications précédentes,
**caractérisée en ce que**
l'armoire de laboratoire (10) comporte plusieurs portes intérieures (18) et les premiers moyens (30) sont réalisés de façon à ce que ces premiers moyens (30) puissent déverrouiller une ou plusieurs portes intérieures (18).
